# EUROPÄISCHE PATENTSCHRIFT

(11) **EP 1 263 333 B1**
(45) Veröffentlichungstag und Bekanntmachung des Hinweises auf die Patenterteilung: **12.04.2006**
(21) Anmeldenummer: 01916897.0
(22) Anmeldetag: 05.03.2001
(51) Int. Cl.: A61B 17/14, B23D 61/12, B27B 19/00

(54) **SÄGEBLATT FÜR MEDIZINISCHE ANWENDUNGEN**
SAW BLADE FOR MEDICAL APPLICATIONS
LAME DE SCIE POUR APPLICATIONS MEDICALES

(30) Priorität: 07.03.2000 DE 10010526
(43) Veröffentlichungstag der Anmeldung: 11.12.2002
(73) Patentinhaber: Hausmann, Thomas, 74564 Crailsheim (DE); Hendrich, Christian, 97084 Würzburg (DE)
(72) Erfinder: Hausmann, Thomas, 74564 Crailsheim (DE); Hendrich, Christian, 97084 Würzburg (DE)
(74) Vertreter: Hufnagel, Walter
(86) Internationale Anmeldenummer: PCT/DE2001/000822
(87) Internationale Veröffentlichungsnummer: WO 2001/066023

(56) Entgegenhaltungen:
- EP-A- 0 695 607
- DE-A- 3 838 844
- FR-A- 1 169 494
- US-A- 4 036 236
- US-A- 5 554 165

## Beschreibung

Die Erfindung bezieht sich auf ein Sägeblatt für medizinische, insbesondere chirurgische, Anwendungen, mit einer Aufnahme zur Aufnahme in eine Säge, die auf das Sägeblatt eine Sägebewegung, insbesondere eine oszillierende Sägebewegung, ausübt.

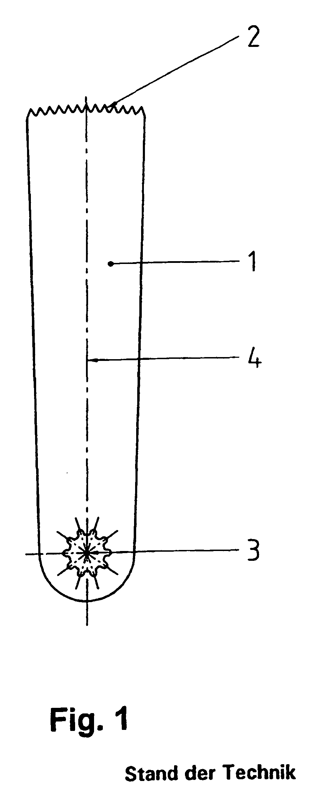

Insbesondere im Bereich der Knie-Chirurgie besteht die Notwendigkeit, Knochen oder Knochenabschnitte sowie anderes Gewebe, wie Knorpel oder dgl. - beispielsweise bei der Implantation eines künstlichen Kniegelenks - mittels einer Säge abzutrennen. Hierzu sind druckluftbetriebene oder elektrische Sägen (häufig als Akku-Geräte ausgebildet) bekannt, die ein Sägeblatt haben, wie es in Fig. 1 dargestellt ist.

Das Sägeblatt (1) ist als dünnes Blech ausgebildet, es hat also eine flächige Struktur. Es weist an einem Ende ein Sägezahnprofil (2) auf, das die Knochen durchtrennt. Das Sägeblatt (1) wird mittels einer Aufnahme (3), beispielsweise mit Mehrzahnprofil, in der Säge aufgenommen. Die Säge übt eine Sägebewegung auf das Sägeblatt aus. Im skizzierten Falle dreht die Säge das Sägeblatt um die Achse der Aufnahme (3) um kleine Winkel hin und her, wodurch sich das Sägezahnprofil (2) oszillierend bewegt. Die Säge übt dabei so große Winkelbewegungen an der Aufnahme (3) auf das Sägeblatt (1) aus, daß die Hubbewegung des Sägezahnprofils (2) nur wenige Millimeter beträgt. Die Sägefrequenz bewegt sich dabei meist in der Größenordnung von ca. 250 Hz.

Gattungsgemäße Sägeblätter sind aus der US 4,036,236 und der EP 0 695 607 A1 bekannt. Bei den dort beschriebenen Sägeblättern ist vorgesehen, daß zwecks Versteifung bzw. zwecks Reduzierung des Gewichts des Sägeblattes Rippen bzw. Nuten angeordnet sind, die linear von der Aufnahme (3, siehe Fig. 1) bis zum Sägezahnprofil (2, siehe Fig. 1) verlaufen.

Bei der Durchtrennung von Knochen kommt es namentlich im Kniebereich darauf an, daß ein möglichst exakter Schnitt ausgeführt wird. Die benötigte Präzision bewegt sich dabei unter einem Millimeter. Die vorbekannten Sägeblätter werfen diesbezüglich Probleme auf:

Das Sägeblatt muß beim Sägen mit einer solch hohen Frequenz oszillieren, daß die dynamischen Kräfte ein Schwingen des Sägeblattes in Richtung der Normalen auf den flächigen Bereich des Sägeblatts bewirken. Im Resonanzbereich kann die Schwingungsamplitude derart groß werden, daß trotz des Einsatzes von Schablonen, die die Säge exakt führen, nicht die benötigte Säge-Präzision eingehalten werden kann. Der Schnitt wird ungenau, was entsprechende Folgen beim chirurgischen Eingriff mit sich bringt.

Ein weiterer Nachteil ist dadurch gegeben, daß die zum Einsatz kommende Säge einer hohen dynamischen Beanspruchung ausgesetzt ist, so daß insbesondere deren Lager einer großen Belastung unterliegen. Bei den üblicherweise zum Einsatz kommenden Sägen ist daher relativ häufig ein Auswechseln von deren Lager notwendig, damit überhaupt eine hinreichende Genauigkeit erzielt werden kann.

Ein Herabsetzen der Dicke des Sägeblattes bringt keine wesentliche Abhilfe. Zwar ist dann der dynamische Effekt infolge der reduzierten Masse des Sägeblattes geringer, allerdings kommt es so zu einer erhöhten Durchbiegung des Blattes beim Aufbringen der Arbeitskräfte.

Der Erfindung liegt daher die **Aufgabe** zugrunde, ein Sägeblatt der gattungsgemäßen Art derart weiter zu entwickeln, daß es ein wesentlich verbessertes dynamisches Verhalten aufweist und trotzdem eine hohe statische Steifigkeit hat. Damit soll es möglich werden, präzise Schnitte durch Knochen durchzuführen. Weiterhin soll die Haltbarkeit der Säge verbessert werden, insbesondere, was deren Lager anbelangt.

Die Lösung dieser Aufgabe durch die Erfindung ist dadurch gekennzeichnet, dass der flächige Bereich des Sägeblattes eine Anzahl an Oberflächen-Einprägungen definierter Kontur aufweist, die in orientierter Anordnung zueinander und entlang einer Geraden ausgerichtet sind, wobei die Gerade in einem Winkel α zur Längsachse des Sägeblattes ausgerichtet ist und der Winkel α zwischen 15° und 45°, vorzugsweise zwischen 25° und 35°, beträgt.

Durch die Einprägungen in die Oberfläche des Sägeblattes wird die Fähigkeit des Blattes beeinflusst, Schwingungen zu transportieren bzw. weiterzuleiten. Das heißt, mit den Einprägungen ist es möglich, das dynamische Verhalten des Sägeblattes zu beeinflussen. Hierdurch kann das Aufschwingverhalten des Sägeblattes im Betrieb wesentlich reduziert werden; die Präzision des Schnittes wird entsprechend erhöht.

Vorzugsweise sind die Oberflächen-Einprägungen auf beiden Seiten des flächigen Bereichs des Sägeblattes angeordnet; namentlich können sie jeweils sich gegenüberliegend auf beiden Seiten des flächigen Bereichs angeordnet sein.

Die Beeinflussung des dynamischen Verhaltens wird durch die Einprägungen besonders günstig beeinflußt, da die Oberflächen-Einprägungen in orientierter Anordnung zueinander und entlang einer Geraden ausgerichtet sind. Die Gerade ist in einem Winkel zur Längsachse des Sägeblattes ausgerichtet.

Für die Kontur der Oberflächen-Einprägungen können verschiedene Geometrien vorgesehen werden. Zunächst kommen Rechtecke in Frage, wobei vorzugsweise die Länge der Rechtecke mindestens das 1,5-fache, insbesondere mindestens das 2-fache, der Breite der Rechtecke beträgt.

Alternativ kommen Oberflächen-Einprägungen in Form von Quadraten, Dreiecken, Kreisen oder Polygonen in Betracht. Dabei betragen die Seitenlängen bzw. die Durchmesser der Oberflächen-Einprägungen mit Vorteil zwischen 1,5 mm und 30 mm, vorzugsweise zwischen 2 mm und 10 mm.

Weiterhin ergeben sich besonders verbesserte dynamische Eigenschaften, wenn die Oberflächen-Einprägungen in äquidistantem Abstand zueinander angeordnet sind; der Abstand zwischen zwei Oberflächen-Einprägungen kann dabei zwischen 0,5 mm und 10 mm, insbesondere zwischen 1 mm und 5 mm, betragen.

Die Einprägetiefe der Oberflächen-Einprägungen beläuft sich bevorzugt auf Werte zwischen 0,05 mm bis 0,5 mm, vorzugsweise zwischen 0,1 mm und 0,2 mm.

Das Sägeblatt besteht weiterbildungsgemäß aus hochfestem Stahl, wobei namentlich an einen hochlegierten Chrom-Stahl gedacht ist. Die Zugfestigkeit des Stahls sollte mindestens 1.200 N/mm² betragen, die Oberflächenhärte des Sägeblattes mindestens 60 HRC (HRC ist die Härte nach Rockwell).

Mit der erfindungsgemäßen Ausgestaltung des Sägeblatts wird erreicht, daß Schwingungen bereits zu Beginn des Aufschaukelns zuverlässig gedämpft werden. So hat das Sägeblatt über den gesamten Frequenzbereich, mit dem es während des Sägevorgangs angeregt wird, keine Neigung, senkrecht zur Blattoberfläche zu schwingen. Die Schnittpräzision wird damit sign:fikant erhöht.

In der Zeichnung sind Ausführungsbeispiele der Erfindung dargestellt.
- Fig. 1: zeigt schematisch die Draufsicht auf ein Sägeblatt gemäß dem Stand der Technik.
- Fig. 2: stellt eine zu Fig. 1 entsprechende Ansicht des Sägeblattes nach einer ersten Ausführung gemäß der Erfindung dar.
- Fig. 3: zeigt den Schnitt A-B gemäß Fig. 2.
- Fig. 4: stellt eine alternative Ausführung zu der Lösung gemäß Fig. 2 dar.
- Fig. 5: zeigt ein Sägeblatt mit einem anderen Grundriß.
- Fig. 6: stellt schließlich ein anderes Sägeblatt für eine Stichsäge dar.

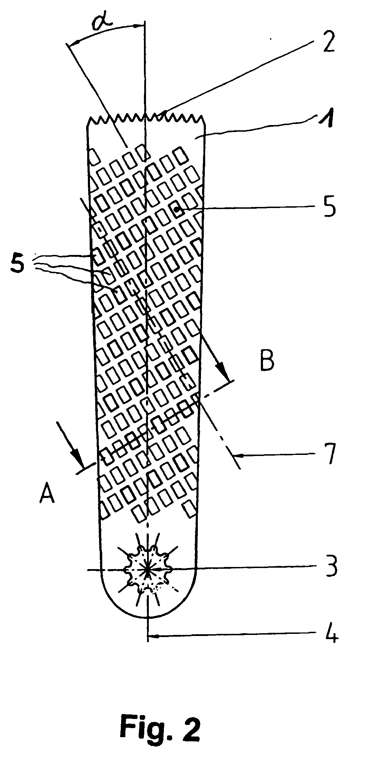

Das in Fig. 2 in der Draufsicht dargestellte Sägeblatt 1 besteht aus einem flachen Grundkörper aus hochlegiertem Chrom-Stahl. Das Sägeblatt 1 weist eine Längsachse 4 auf. Am einen Ende ist das Sägeblatt 1 mit einem Sägezahnprofil 2 versehen, das beispielsweise mit einem Laserschneidprozeß eingebracht ist. Am anderen Ende ist eine Aufnahme 3 eingearbeitet, die vorliegend als Vielzahnprofil ausgebildet ist, das mit einem entsprechenden Gegenstück der - nicht dargestellten - Säge zusammenwirkt.

Die Säge dreht das Sägeblatt 1 im Betrieb um den Mittelpunkt der Aufnahme 3 in der Ebene des flächigen Bereichs, wodurch das Sägezahnprofil 2 hin- und herbewegt wird und so Knochen und anderes Gewebe durchtrennen kann.

Zur Verbesserung des dynamischen Verhaltens ist die Oberfläche des Sägeblattes mit einer Vielzahl von Oberflächen-Einprägungen 5 versehen. Im Ausführungsbeispiel gemäß Fig. 2 sind die Einprägungen 5 in Form kleiner Rechtecke eingebracht.

Wie zu erkennen ist, haben die einzelnen Rechtecke dabei eine zueinander orientierte Ausrichtung. Sie sind in geringem Abstand hintereinander entlang einer Geraden 7 positioniert, die gestrichelt in Fig. 2 eingetragen ist. Die Gerade 7 wiederum ist in einem Winkel α zur Längsachse 4 angeordnet. Der Winkel α beträgt dabei bevorzugt 30 °.

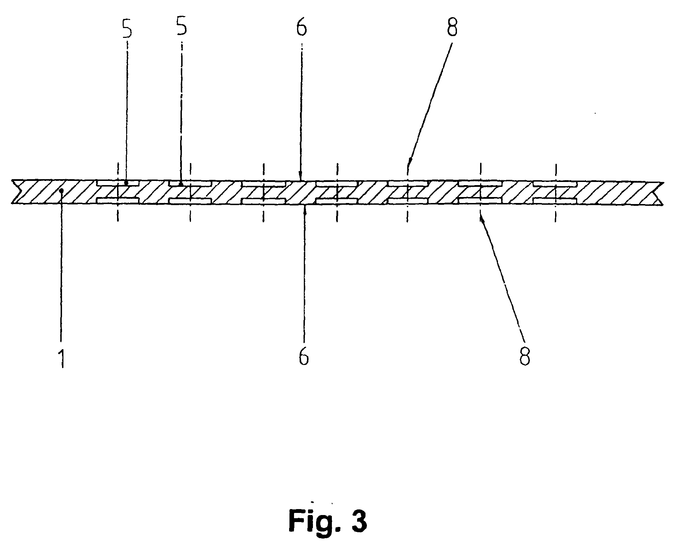

Wie bereits erwähnt, handelt es sich bei den eingebrachten Elementen 5 um Einprägungen an der Oberfläche des Sägeblattes 1. Dies geht gut sichtbar aus Fig. 3 hervor. Dort ist der Schnitt A-B gemäß Fig. 2 dargestellt. Mit einem - nicht dargestellten - Prägewerkzeug sind die Einprägungen 5 beidseitig der flächigen Bereiche 6 eingeprägt worden, wobei sich die jeweiligen Einprägungen 5 auf beiden Seiten des Sägeblattes 1 gegenüberliegen. Hierdurch wird erreicht, daß sich zwischen den Einprägungen 5 ein verdichteter bzw. komprimierter Materialbereich 8 bildet, der infolge der Materialverdichtung die Eigenschaft hat, schwingungsbedingte Anregungen abzulenken. Durch die spezielle Anordnung der Einprägungen 5 entlang der winkelig ausgerichteten Geraden 7 (siehe Fig. 2) kommt es zur Ablenkung der Schwingungsanregung "aus dem Sägeblatt heraus", weshalb das dynamische Verhalten des Sägeblattes 1 signifikant verbessert ist.

Die Einprägungen 5 können auch in einer alternativen Form ausgebildet sein.

Statt der rechteckigen Querschnittsform in Fig. 3 können auch wannenförmige oder ähnlich ausgeführte Einprägungen vorgesehen werden, die einen kontinuierlichen Materialübergang vom flächigen Bereich 6 in den Einprägungsbereich ermöglichen. In diesem Falle können die Einprägungen 5 unter Einsatz eines balligen Prägestempels hergestellt werden.

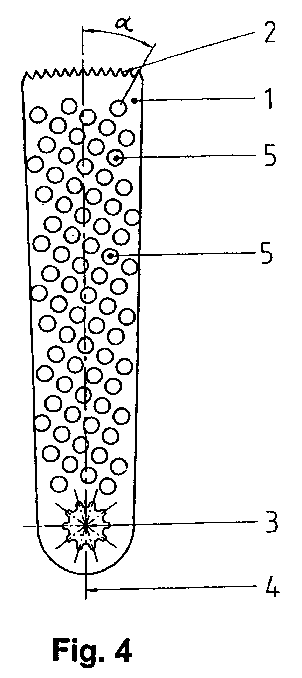

Die Fig. 4 zeigt eine Variante zur Ausgestaltung gemäß Fig. 2. Hier sind die Einprägungen 5 in Form von Kreisen ausgebildet. Die Kreise sind wiederum entlang einer Geraden ausgerichtet, die um den Winkel α zur Achse 4 ausgerichtet ist.

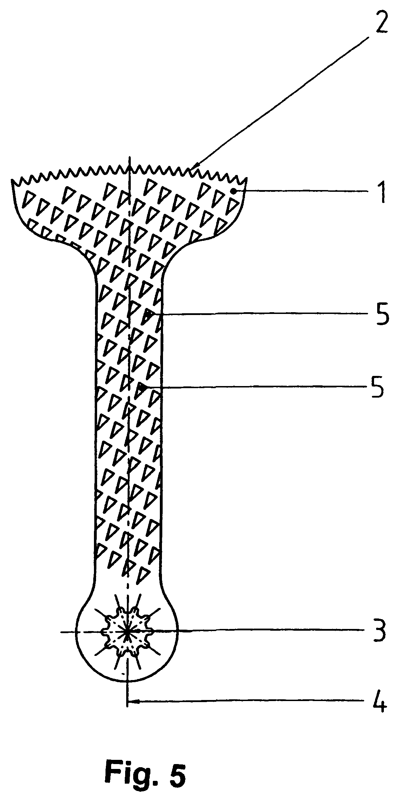

In Fig. 5 ist zu sehen, daß das Erfindungskonzept auch auf andere Grundkonturen des Sägeblattes 1 angewendet werden kann. Vorliegend ist das Sägeblatt 1 im Bereich des Sägezahnprofils 2 hammerkopfartig ausgeführt.

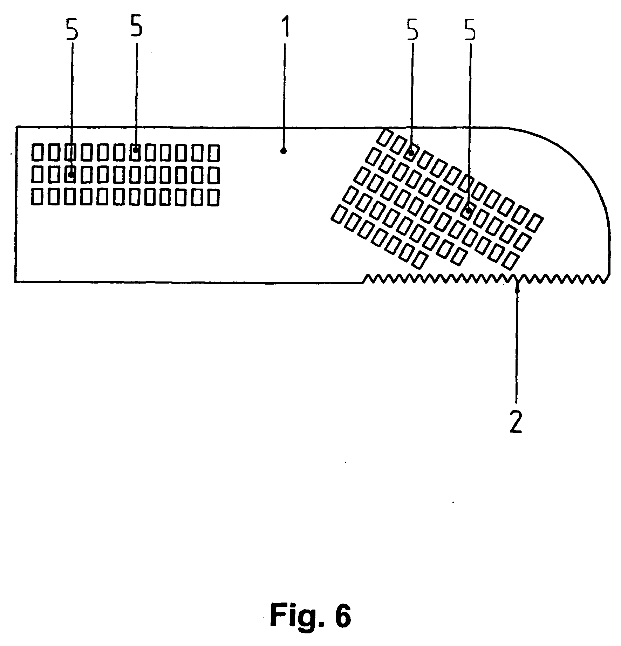

Das Erfindungskonzept läßt sich weiterhin auch auf andere Typen medizinischer Sägen anwenden, wie es aus Fig. 6 hervorgeht. Dort ist ein Sägeblatt 1 zu sehen, das in einer Stichsäge zum Einsatz kommt. Die Oberflächen-Einprägungen 5 sind auch hier orientiert ausgerichtet, so daß sich aufbauende Schwingungen aus dem Sägeblatt 1 abgelenkt werden. Auch hier ergibt sich ein wesentlich verbessertes dynamisches Verhalten des Sägeblattes 1 im Betrieb.

Die Oberflächen-Einprägungen 5 können rationell durch einen Prägeprozeß eingebracht werden. Das Sägeblatt 1 ist so wirtschaftlich herstellbar.

## Patentansprüche

1. Sägeblatt (1) für medizinische, insbesondere chirurgische Anwendungen mit einer Aufnahme (3) zur Aufnahme in eine Säge, die auf das Sägeblatt (1) eine Sägebewegung, insbesondere eine oszillierende Sägebewegung ausübt, wobei der flächige Bereich (6) des Sägeblattes (1) eine Anzahl an Oberflächen-Einprägungen (5) definierter Kontur aufweist, die in orientierter Anordnung zueinander und entlang einer Geraden ausgerichtet sind, wobei die Gerade in einem Winkel (α) zur Längsachse (4) des Sägeblattes (1) ausgerichtet ist und **dadurch gekennzeichnet, dass** der Winkel (α) zwischen 15° und 45°, vorzugsweise zwischen 25° und 35°, beträgt.

2. Sägeblatt (1) nach Anspruch 1, **dadurch gekennzeichnet, dass** die Oberflächen-Einprägungen (5) auf beiden Seiten des flächigen Bereichs (6) des Sägeblattes (1) angeordnet sind.

3. Sägeblatt (1) nach Anspruch 1 oder 2, **dadurch gekennzeichnet, dass** die Oberflächen-Einprägungen (5) jeweils sich gegenüberliegend auf beiden Seiten des flächigen Bereichs (6) des Sägeblattes (1) angeordnet sind.

4. Sägeblatt (1) nach einem der Ansprüche 1 bis 3, **dadurch gekennzeichnet, dass** die Oberflächen-Einprägungen (5) die Kontur von Rechtecken haben.

5. Sägeblatt (1) nach Anspruch 4, **dadurch gekennzeichnet, dass** die Länge der Rechtecke mindestens das 1,5-fache, vorzugsweise mindestens das 2-fache, der Breite der Rechtecke beträgt.

6. Sägeblatt (1) nach einem der Ansprüche 1 bis 3, **dadurch gekennzeichnet, dass** die Oberflächen-Einprägungen (5) die Kontur von Quadraten haben.

7. Sägeblatt (1) nach einem der Ansprüche 1 bis 3, **dadurch gekennzeichnet, dass** die Oberflächen-Einprägungen (5) die Kontur von Dreiecken haben.

8. Sägeblatt (1) nach einem der Ansprüche 1 bis 3, **dadurch gekennzeichnet, dass** die Oberflächen-Einprägungen (5) die Kontur von Kreisen haben.

9. Sägeblatt (1) nach einem der Ansprüche 1 bis 3, **dadurch gekennzeichnet, dass** die Oberflächen-Einprägungen (5) die Kontur von Polygonen haben.

10. Sägeblatt (1) nach einem der Ansprüche 4 bis 9, **dadurch gekennzeichnet dass** die Seitenlängen bzw. die Durchmesser der Oberflächen-Einprägungen (5) zwischen 1,5 mm und 30 mm, vorzugsweise zwischen 2 mm und 10 mm, betragen.

11. Sägeblatt (1) nach einem der Ansprüche 1 bis 10, **dadurch gekennzeichnet, dass** die Oberflächen-Einprägungen (5) in äquidistantem Abstand zueinander angeordnet sind.

12. Sägeblatt (1) nach einem der Ansprüche 1 bis 11, **dadurch gekennzeichnet, dass** der Abstand zwischen zwei Oberflächen-Einprägungen (5) zwischen 0,5 mm und 10 mm, vorzugsweise 1 mm und 5 mm, beträgt.

13. Sägeblatt (1) nach einem der Ansprüche 1 bis 12, **dadurch gekennzeichnet, dass** die Einprägetiefe der Oberflächen-Einprägungen (5) zwischen 0,05 mm bis 0,5 mm, vorzugsweise zwischen 0,1 mm und 0,2 mm, beträgt.

14. Sägeblatt (1) nach einem der Ansprüche 1 bis 13, **dadurch gekennzeichnet, dass** das Sägeblatt (1) aus hochfestem Stahl besteht.

15. Sägeblatt (1) nach Anspruch 14, **dadurch gekennzeichnet, dass** der Stahl ein hochlegierter Chrom-Stahl ist.

16. Sägeblatt (1) nach Anspruch 14 oder 15, **dadurch gekennzeichnet, dass** die Zugfestigkeit des Stahls mindestens 1.200 N/mm² beträgt.

17. Sägeblatt (1) nach einem der Ansprüche 1 bis 16, **dadurch gekennzeichnet, dass** die Oberflächenhärte des Sägeblattes (1) mindestens 60 HRC beträgt.

## Claims

1. Saw blade (1) for medical, in particular surgical applications with a mounting (3) for mounting in a saw which exerts a sawing motion, in particular an oscillating sawing motion, on the saw blade (1), wherein the areal region (6) of the saw blade (1) exhibits a number of surface impressions (5) of defined contour which are aligned in an oriented arrangement relative to one another and along a straight line, the straight line being aligned at an angle (α) to the longitudinal axis (4) of the saw blade (1), **characterised in that** the angle (α) is between 15° and 45°, preferably between 25° and 35°.

2. Saw blade (1) according to claim 1, **characterised in that** the surface impressions (5) are arranged on both sides of the areal region (6) of the saw blade (1).

3. Saw blade (1) according to claim 1 or 2, **characterised in that** the surface impressions (5) are in each case arranged facing one another on both sides of the areal region (6) of the saw blade (1).

4. Saw blade (1) according to one of claims 1 to 3, **characterised in that** the surface impressions (5) have the contour of rectangles.

5. Saw blade (1) according to claim 4, **characterised in that** the length of the rectangles is at least 1.5 times, preferably at least 2 times, the width of the rectangles.

6. Saw blade (1) according to one of claims 1 to 3, **characterised in that** the surface impressions (5) have the contour of squares.

7. Saw blade (1) according to one of claims 1 to 3, **characterised in that** the surface impressions (5) have the contour of triangles.

8. Saw blade (1) according to one of claims 1 to 3, **characterised in that** the surface impressions (5) have the contour of circles.

9. Saw blade (1) according to one of claims 1 to 3, **characterised in that** the surface impressions (5) have the contour of polygons.

10. Saw blade (1) according to one of claims 4 to 9, **characterised in that** the side lengths or diameters of the surface impressions (5) are between 1.5 mm and 30 mm, preferably between 2 mm and 10 mm.

11. Saw blade (1) according to one of claims 1 to 10, **characterised in that** the surface impressions (5) are arranged equidistant from one another.

12. Saw blade (1) according to one of claims 1 to 11, **characterised in that** the distance between two surface impressions (5) is between 0.5 mm and 10 mm, preferably 1 mm and 5 mm.

13. Saw blade (1) according to one of claims 1 to 12, **characterised in that** the depth of impression of the surface impressions (5) is between 0.05 mm and 0.5 mm, preferably between 0.1 mm and 0.2 mm.

14. Saw blade (1) according to one of claims 1 to 13, **characterised in that** the saw blade (1) is made of high tensile steel.

15. Saw blade (1) according to claim 14, **characterised in that** the steel is a high alloyed chromium steel.

16. Saw blade (1) according to claim 14 or 15, **characterised in that** the tensile strength of the steel is at least 1200 N/mm².

17. Saw blade (1) according to one of claims 1 to 16, **characterised in that** the surface hardness of the saw blade (1) is at least 60 HRC.

## Revendications

1. Lame de scie (1) pour applications médicales, notamment chirurgicales, comprenant un logement (3) pour être logée dans une scie qui exerce sur la lame de scie (1)un mouvement de sciage, notamment oscillant, la zone plane (6) de la lame de scie (1) présentant un certain nombre d'empreintes de surface (5) de contour bien défini et orientées les unes par rapport aux autres le long d'une droite, la droite étant orientée suivant un angle (a) défini par rapport à l'axe longitudinal (4) de la lame de scie (1),
**caractérisée en ce que**
l'angle (α) est compris entre 15° et 45°, de préférence entre 25° et 35°.

2. Lame de scie (1) selon la revendication 1,
**caractérisée en ce que**
les empreintes de surface (5) sont disposées des deux côtés de la zone plane (6) de la lame de scie (1).

3. Lame de scie (1) selon la revendication 1 ou 2,
**caractérisée en ce que**
les empreintes de surface (5) sont disposées à chaque de fois de manière opposée les unes par rapport aux autres des deux côtés de la zone plane (6) de la lame de scie (1).

4. Lame de scie (1) selon l'une des revendications 1 à 3,
**caractérisée en ce que**
les empreintes de surface (5) présentent un contour en forme de rectangle.

5. Lame de scie (1) selon la revendication 4,
**caractérisée en ce que**
la longueur des rectangles représente au moins 1,5 fois la largeur des rectangle, et de préférence au moins 2 fois cette largeur.

6. Lame de scie (1) selon l'une des revendications 1 à 3,
**caractérisée en ce que**
les empreintes de surface (5) présentent un contour en forme de carré.

7. Lame de scie selon l'une des revendications 1 à 3,
**caractérisée en ce que**
les empreintes de surface (5) présentent un contour en forme de triangle.

8. Lame de scie (1) selon l'une des revendications 1 à 3,
**caractérisée en ce que**
les empreintes de surface (5) présentent un contour en forme de cercle.

9. Lame de scie (1) selon l'une des revendications 1 à 3,
**caractérisée en ce que**
les empreintes de surface (5) présentent un contour en forme de polygone.

10. Lame de scie (1) selon l'une des revendications 4 à 9,
**caractérisée en ce que**
les longueurs latérales ou les diamètres des empreintes de surface (5) sont compris(es) entre 1,5 mm et 30 mm, de préférence entre 2 mm et 10 mm.

11. Lame de scie (1) selon l'une des revendications 1 à 10,
**caractérisée en ce que**
les empreintes de surface (5) sont disposées à équidistance les unes par rapport aux autres.

12. Lame de scie (1) selon l'une des revendications 1 à 11,
**caractérisée en ce que**
la distance entre deux empreintes de surface (5) est comprise entre 0,5 mm et 10 mm, de préférence entre 1 mm et 5 mm.

13. Lame de scie (1) selon l'une des revendications 1 à 12,
**caractérisé en ce que**
la profondeur des empreintes de surface (5) est comprise entre 0,05 mm et 0,5 mm, de préférence entre 0,1 mm et 0,2 mm.

14. Lame de scie (1) selon l'une des revendications 1 à 13,
**caractérisée en ce que**
la lame de scie (1) est composée d'acier à forte résistance.

15. Lame de scie (1) selon la revendication 14,
**caractérisée en ce que**
l'acier est un acier à haute teneur en chrome.

16. Lame de scie (1) selon la revendication 14 ou 15,
**caractérisée en ce que**
l'acier présente une résistance à la traction de 1200 N/mm².

17. Lame de scie (1) selon l'une des revendications 1 à 16,
**caractérisée en ce que**
la dureté de surface de la lame de scie (1) est au minimum de 60 HRC.
